# FASCICULE DE BREVET EUROPEEN

(11) **EP 2 047 879 B1**
(45) Date de publication et mention de la délivrance du brevet: **13.02.2013**
(21) Numéro de dépôt: 08166451.8
(22) Date de dépôt: 13.10.2008
(51) Int. Cl.: A61M 5/32

(54) **Dispositif de sécurité pour une seringue d'injection de liquide et ensemble à seringue comprenant ce dispositif**
Sicherheitseinrichtung für eine Spritzvorrichtung
Safety assembly for an injection syringe

(30) Priorité: 11.10.2007 FR 0758231; 23.10.2007 US 960978 P
(43) Date de publication de la demande: 15.04.2009
(73) Titulaire: Rexam Pharma La Verpillière, 38290 La Verpillière Cedex (FR)
(72) Inventeur: Brand, Julien, 38110, SAINT CLAIR DE LA TOUR (FR); Dugand, Pascal, 38780, ESTRABLIN (FR); Lanzi, Sylvain, 38850, CHIRENS (FR); Rimlinger, Thierry, 38080, L'ISLE D'ABEAU (FR)
(74) Mandataire: Thiollier, Clémence-Olivia Laure Marie

(56) Documents cités:
- WO-A-03/047664
- WO-A-03/077977
- WO-A-2005/089831
- WO-A-2007/109352
- US-A1- 2004 127 857

## Description

La présente invention concerne le domaine des dispositifs de sécurité pour seringue d'injection de liquide, notamment pour seringue pré-remplie.

On connaît déjà dans l'état de la technique, notamment d'après WO 2006/050304, une seringue d'injection de liquide du type comprenant un corps de forme générale tubulaire formant un réservoir pour le liquide sur lequel est rapporté un embout de forme générale annulaire pour la fixation d'un porte-aiguille sur le corps de seringue.

L'embout de fixation est parfois désigné par les termes anglais "luer lock" et le porte-aiguille est parfois désigné par les termes anglais "luer needle".

La seringue comprend également un poussoir monté déplaçable axialement dans le corps entre une position d'attente et une position de fin d'injection de liquide.

Dans ce qui suit un élément sera qualifié de proximal ou de distal selon qu'il est proche ou éloigné axialement de l'extrémité du poussoir destinée à être actionnée par un utilisateur.

WO 2006/050304 décrit également un dispositif de sécurité, pour une seringue du type précité, ce dispositif étant du type comportant :
- un premier organe de forme générale tubulaire, que l'on appellera par la suite fourreau de protection, et
- un second organe de forme générale tubulaire, que l'on appellera par la suite support de seringue, logé dans le fourreau sensiblement coaxialement à ce dernier.

Les fourreau de protection et support de seringue sont déplaçables axialement l'un par rapport à l'autre entre deux positions que l'on appellera par la suite position de dégagement de l'aiguille de seringue et position d'escamotage de cette aiguille.

Le dispositif de sécurité permet un escamotage automatique de l'aiguille dans le fourreau afin d'éviter qu'une personne manipulant l'ensemble de seringue après son utilisation normale, c'est-à-dire après injection du liquide dans le corps d'un patient, ne se pique accidentellement avec l'aiguille.

L'embout de fixation comporte une extrémité proximale généralement emmanchée sur une extrémité distale du corps de la seringue en forme générale de cône.

Par ailleurs, l'embout de fixation comporte une extrémité distale destinée à coopérer par vissage avec un porte-aiguille. Ce porte-aiguille et l'aiguille qu'il porte sont généralement recouverts, avant utilisation de la seringue, par un capuchon. Le cas échéant, avant mise en place du porte-aiguille sur le corps de la seringue, un bouchon d'obturation du corps de la seringue est vissé sur l'embout de fixation.

Le couple nécessaire au dévissage du bouchon ou au vissage du porte-aiguille est susceptible d'entraîner en rotation l'embout de fixation autour de l'axe du corps de la seringue. Il convient donc de pouvoir s'opposer à la rotation de l'embout de fixation pour parvenir notamment à dévisser le bouchon ou visser le porte-aiguille.

Or, après montage de la seringue dans le dispositif de sécurité, l'embout de fixation est généralement rendu pratiquement inaccessible par le fourreau de protection qui le recouvre. L'utilisateur ne peut donc pas agir directement sur l'embout de fixation pour l'immobiliser en rotation notamment lors du dévissage du bouchon ou du vissage du porte-aiguille.

WO 2006/050304 propose des moyens d'immobilisation en rotation de l'embout de fixation comprenant notamment une paire de languettes ménagées dans le fourreau de protection et dans le support de seringue. En considérant les fourreau et support dans leur position relative de dégagement de l'aiguille de seringue, la languette du fourreau recouvre la languette du support de seringue. Les languettes sont déformables élastiquement radialement. Pour immobiliser en rotation l'embout de fixation, l'utilisateur appuie sur la languette du fourreau de façon à l'enfoncer radialement vers l'axe de ce fourreau, ce qui a pour effet d'enfoncer la languette du support (recouverte par la languette du fourreau) jusqu'au contact avec l'embout de fixation.

L'invention a pour but de proposer des moyens d'immobilisation en rotation de l'embout de fixation plus simples que ceux décrits dans WO 2006/050304, ces moyens d'immobilisation étant susceptibles d'être activés sans que l'utilisateur n'ait à effectuer un geste spécifique pour l'obtention de cette immobilisation.

A cet effet, l'invention a pour objet un dispositif de sécurité pour une seringue d'injection de liquide comprenant un corps de forme générale tubulaire formant un réservoir pour le liquide sur lequel est rapporté un embout de forme générale annulaire pour la fixation d'un porte-aiguille sur le corps de seringue,

le dispositif étant du type comportant :
- un premier organe de forme générale tubulaire, appelé fourreau de protection, et
- un second organe de forme générale tubulaire, appelé support de seringue, logé dans le fourreau sensiblement coaxialement à ce dernier,
les fourreau et support étant déplaçables axialement l'un par rapport à l'autre entre deux positions dites de dégagement de l'aiguille de seringue et d'escamotage de cette aiguille,
**caractérisé en ce que :**
- le support de seringue comprend des premiers moyens de solidarisation en rotation autour de son axe comprenant au moins un cliquet rappelé élastiquement dans une position dans laquelle ce cliquet est destiné à coopérer avec des moyens complémentaires de solidarisation en rotation portés par l'embout de fixation, et
- le fourreau de protection et le support de seringue comprennent des seconds moyens complémentaires de solidarisation en rotation entre eux autour de leurs axes, ces seconds moyens de solidarisation en rotation étant activés au moins lorsque les fourreau et support sont dans leur position relative de dégagement de l'aiguille.

Les premiers moyens de solidarisation en rotation sont simples et efficaces.

En effet, on considère par exemple qu'un bouchon d'obturation de la seringue est vissé sur l'embout de fixation. Grâce au cliquet, cet embout de fixation est solidarisé en rotation avec le support de seringue, soit avant même qu'un utilisateur dévisse le bouchon, soit après une brève course de dévissage de ce bouchon. Par ailleurs, les seconds moyens complémentaires de solidarisation en rotation liant en rotation le support de seringue avec le fourreau, l'embout de fixation est, par transitivité, solidarisé en rotation avec le fourreau. Ainsi, un utilisateur saisissant le fourreau peut facilement dévisser le bouchon porté par l'embout de fixation puis visser un porte-aiguille sur cet embout de fixation, sans que ces opérations de vissage et dévissage soient empêchées par une rotation intempestive de l'embout de fixation.

Un dispositif de sécurité selon l'invention peut comporter également au moins l'une des caractéristiques optionnelles suivantes :
- le support de seringue porte au moins deux cliquets de solidarisation en rotation, diamétralement opposés ;
- le cliquet est formé par une patte axiale venue de matière avec le support de seringue rappelée élastiquement radialement vers l'axe de ce support de seringue, destinée à coopérer avec une rainure complémentaire de solidarisation en rotation ménagée sur le contour de l'embout de fixation ;
- le fourreau comprend au moins un dégagement radial qui, lorsque les fourreau et support sont dans leur position relative de dégagement de l'aiguille de seringue, est au droit radialement de la patte axiale de solidarisation en rotation pour autoriser le déplacement radial de cette patte à l'encontre de sa force de rappel ;
- la patte axiale de solidarisation en rotation étant portée par une extrémité distale du support de seringue, le dégagement radial est formé par une fente axiale ménagée dans une extrémité distale du fourreau ;
- les seconds moyens complémentaires de solidarisation en rotation comprennent au moins une paire de saillie et rainure complémentaires ménagées sur les support de seringue et fourreau de protection ;
- la saillie des seconds moyens de solidarisation en rotation est ménagée sur une extrémité proximale du support de seringue et la rainure des seconds moyens de solidarisation en rotation est formée par une fente ménagée dans une extrémité proximale du fourreau de protection ;
- les seconds moyens complémentaires de solidarisation en rotation comprennent deux paires de saillie et rainure complémentaires diamétralement opposées ;
- le dispositif comprend des troisièmes moyens complémentaires de solidarisation en rotation du support par rapport au fourreau autour de leurs axes, ces troisièmes moyens étant activés au moins lors du déplacement axial des fourreau et support l'un par rapport à l'autre de la position de dégagement de l'aiguille de seringue vers celle d'escamotage de cette aiguille. Ces troisièmes moyens permettent notamment d'éviter la rotation du support par rapport au fourreau au cours de la détente d'un ressort.

L'invention a également pour objet un ensemble à seringue, du type comprenant :
- une seringue d'injection de liquide comprenant un corps de forme générale tubulaire formant un réservoir pour le liquide sur lequel est rapporté un embout de forme générale annulaire pour la fixation d'un porte-aiguille sur le corps de seringue, et
- un dispositif de sécurité pour la seringue,
caractérisé en ce que le dispositif de sécurité est tel que défini ci-dessus.

Un ensemble selon l'invention peut comporter également au moins l'une des caractéristiques optionnelles suivantes :
- le contour de l'embout de fixation à une forme générale crénelée délimitant plusieurs rainures axiales de solidarisation en rotation réparties angulairement ;
- l'embout de fixation est destiné à coopérer par vissage avec un bouchon d'obturation de la seringue ou le porte-aiguille.

L'invention sera mieux comprise à la lecture de la description qui va suivre, donnée uniquement à titre d'exemple et faite en se référant aux dessins dans lesquels :
- la figure 1 est une vue en coupe axiale d'un ensemble à seringue selon l'invention, la seringue étant munie d'un embout de fixation sur lequel est vissé un bouchon d'obturation de la seringue ;
- la figure 2 est une vue en coupe suivant la ligne 2-2 de la figure 1, dans laquelle le bouchon d'obturation a été remplacé par un porte-aiguille vissé sur l'embout de fixation, l'ensemble étant dans une configuration précédant l'utilisation de la seringue ;
- la figure 3 est une vue similaire à la figure 2, l'ensemble étant dans une configuration après utilisation de la seringue ;
- la figure 4 est une section, à échelle agrandie, suivant la ligne 4-4 de la figure 1 ;
- la figure 5 est une vue en perspective d'une partie de l'ensemble à seringue illustré sur les figures 1 à 3 comprenant notamment le support de seringue et l'embout de fixation de porte-aiguille ;
- la figure 6 est une vue de détail de la partie cerclée 6 de la figure 5 montrant en plus, par rapport à cette figure 5, un bouchon d'obturation de la seringue vissé sur l'embout de fixation ;
- la figure 7 est une vue similaire à la figure 6 montrant en plus, par rapport à cette figure 6, le fourreau de protection recouvrant le support de seringue ; et
- la figure 8 est une vue similaire à la figure 6 montrant l'embout de fixation dans une position angulaire par rapport au support de seringue différente de celle représentée sur la figure 6,
- la figure 9 est une vue en perspective du support de seringue de l'ensemble à seringue illustré sur les figures 1 à 3 selon deux variantes de l'invention.

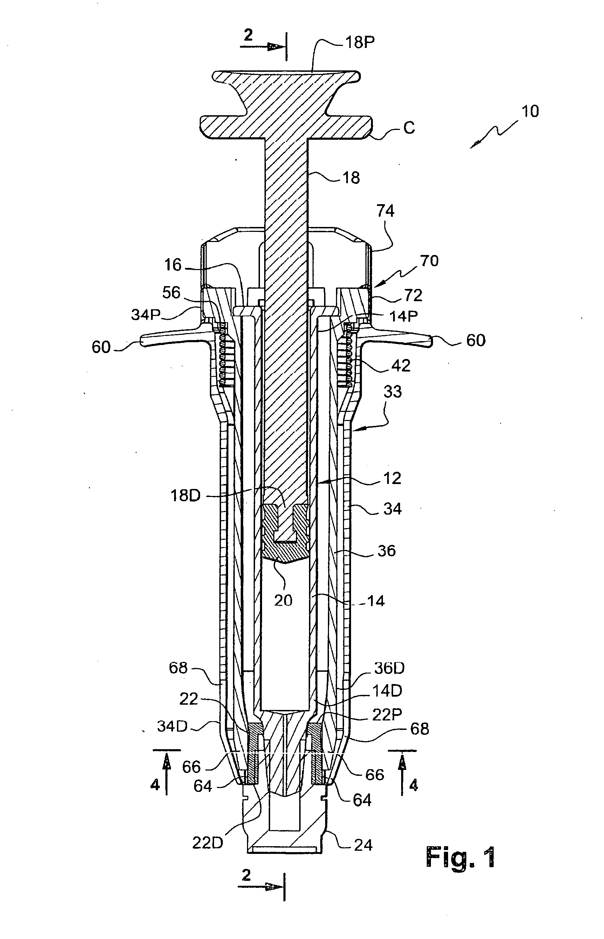

On a représenté sur les figures 1 à 3 un ensemble à seringue, selon l'invention, désigné par la référence générale 10.

L'ensemble 10 comprend une seringue classique 12 destinée à l'injection d'un liquide, notamment d'un liquide médical.

La seringue 12 comprend un corps tubulaire 14 de seringue formant un réservoir pour le liquide. Le corps 14 comprend une extrémité proximale 14P ouverte, munie d'une collerette 16, et une extrémité distale 14D en forme générale de cône convergeant à l'opposé de l'extrémité proximale 14P.

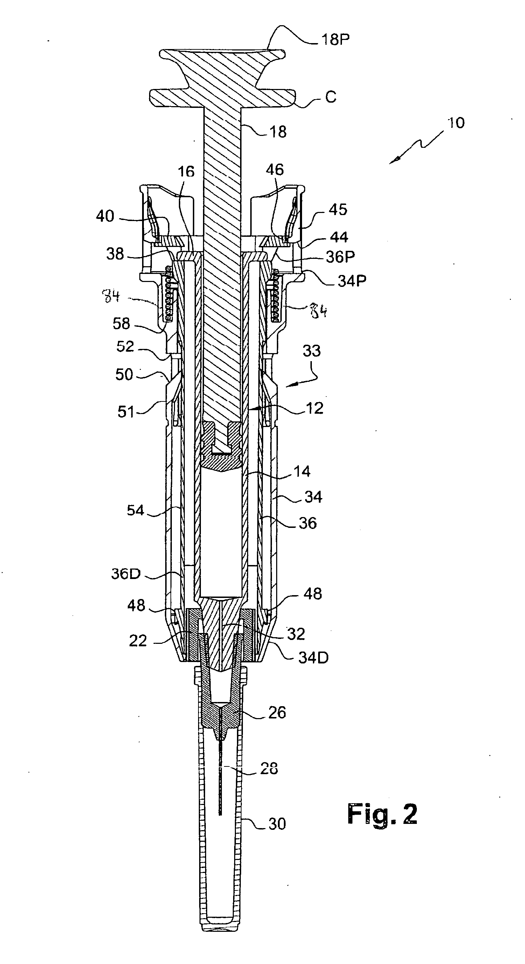

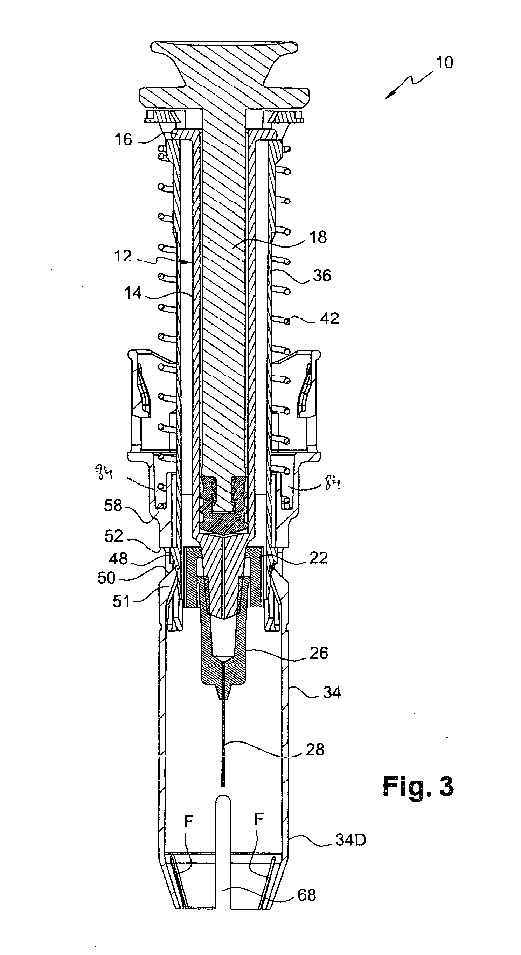

La seringue 12 comprend également un poussoir 18 monté déplaçable axialement dans le corps 14 entre une position d'attente, tel que représenté sur les figures 1 et 2, et une position de fin d'injection de liquide, tel que représenté sur la figure 3.

Le poussoir 18, fabriqué par exemple en plastique, comporte une extrémité proximale 18P, externe au corps 14, et une extrémité distale 18D, interne au corps 14, portant un piston classique 20. L'extrémité proximale 18P du poussoir forme une extrémité de manoeuvre de ce poussoir.

La seringue 12 comprend également un embout 22 de fixation d'un bouchon 24 d'obturation de la seringue, tel que représenté sur la figure 1, ou d'un porte-aiguille 26 tel que représenté sur les figures 2 et 3.

L'embout de fixation 22, de forme générale annulaire, comporte une extrémité proximale 22P emmanchée de façon connue en soi sur une surface complémentaire de l'extrémité distale 14D du corps 14. L'embout de fixation 22 comporte également une extrémité distale 22D munie d'une surface interne filetée destinée à coopérer par vissage avec une surface filetée complémentaire du bouchon d'obturation 24 ou du porte-aiguille 26.

Le porte-aiguille 26 porte une aiguille 28 susceptible d'être protégée par un capuchon amovible 30 emmanché sur le porte-aiguille 26, comme cela est représenté sur la figure 2.

Un canal axial 32, ménagé dans l'extrémité distale 14D du corps 14 de seringue, permet le passage de liquide entre ce corps 14 et le porte-aiguille 26.

L'ensemble 10 comprend également un dispositif de sécurité 33 selon l'invention.

Le dispositif 33 comprend un premier organe de forme générale tubulaire, appelé fourreau de protection 34, et un second organe de forme générale tubulaire, appelé support 36 de seringue. Ce support 36 est logé dans le fourreau 34 sensiblement coaxialement à ce dernier.

Les fourreau 34 et support 36 sont fabriqués par exemple en plastique.

Les fourreau 34 et support 36 sont déplaçables axialement l'un par rapport à l'autre entre une première position, dite de dégagement de l'aiguille, tel que représenté sur les figures 1 et 2, et une seconde position, dite d'escamotage de l'aiguille, tel que représenté sur la figure 3.

Le corps 14 de seringue est logé de façon connue en soi dans le support 36. Plus particulièrement, le corps 14 de seringue est immobilisé axialement (éventuellement avec jeu) dans le support 36 de seringue par encliquetage de la collerette 16 entre un siège d'appui 38 ménagé dans le support 36 et au moins une butée de verrouillage 40 escamotable solidaire du support 36 (voir notamment figure 2). Dans l'exemple décrit, le support 36 comporte deux butées de verrouillage 40 diamétralement opposées.

Le cas échéant les jeux radial et transversal de la collerette 16 dans le logement formé par le siège 38 et les butées 40 peut être limité par coopération de formes complémentaires.

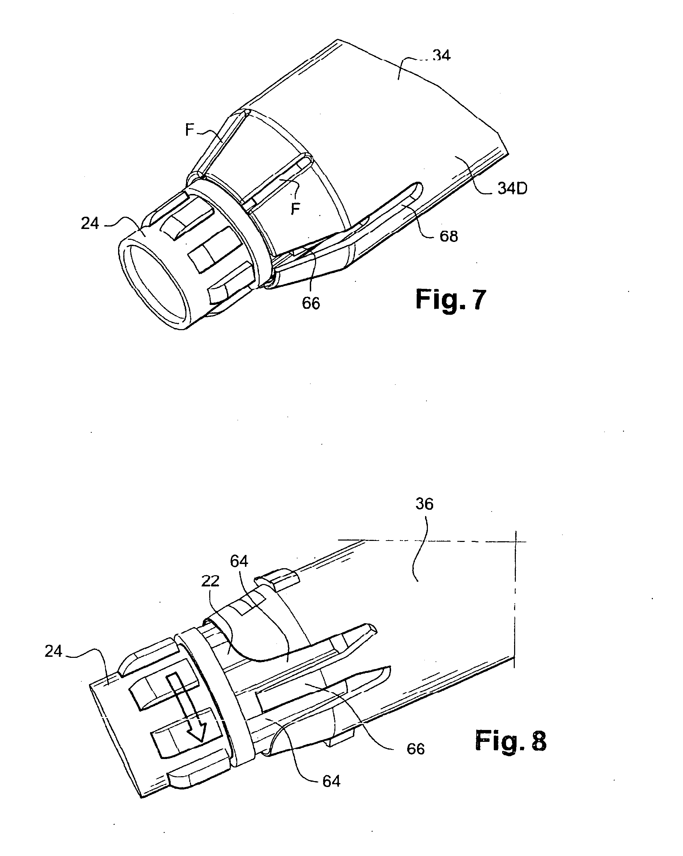

On notera que le fourreau de protection 34 est muni d'une extrémité distale 34D de forme générale convergente en s'éloignant de l'extrémité proximale 34P de ce fourreau (voir notamment figure 7). Cette extrémité distale 34D est munie d'ouvertures ou fentes axiales F autorisant la déformation élastique radiale de cette extrémité distale 34D de façon notamment à autoriser une variation élastique du diamètre de l'ouverture débouchante de l'extrémité distale 34D et ainsi autoriser le passage d'éléments de l'ensemble 10 de diamètres variés à travers cette ouverture.

Par ailleurs, le dispositif de sécurité 33 comprend des moyens de retenue du support 36 en position de dégagement d'aiguille par rapport au fourreau 34. Ces moyens de retenue s'opposent à la force élastique de moyens de rappel comprenant un ressort de poussée 42. Comme cela est illustré sur la figure 2, on voit que, dans la position de dégagement de l'aiguille, le ressort 42 est logé dans une gorge 84 de forme générale annulaire, ménagée dans cet exemple dans l'extrémité proximale 34P du fourreau 34. Cette gorge 84 assure également un positionnement axial et radial du ressort 42.

En se référant à la figure 2, on voit que, dans l'exemple illustré, les moyens de retenue comprennent au moins une paire de butées de retenue complémentaires 44, 46. Une première butée de retenue 44 est formée par l'extrémité libre d'une languette axiale 45 ménagée dans une extrémité proximale 34P du fourreau 34. Cette languette 45 est déformable élastiquement radialement. La seconde butée de retenue 46 est ménagée sur une extrémité proximale 36P du support 36 de seringue. De préférence, le dispositif de sécurité 33 comporte deux paires de butées de retenue 44, 46 diamétralement opposées.

Le dispositif de sécurité 33 comprend également des moyens de verrouillage du fourreau 34 en position d'escamotage de l'aiguille 28 tel que représenté sur la figure 3.

En se référant aux figures 2 et 3, on voit que, dans l'exemple illustré, les moyens de verrouillage comprennent au moins une saillie radiale 48 ménagée dans une extrémité distale 36D du support 36 de seringue destinée à s'encliqueter entre une paire de butées d'immobilisation axiale 50, 52. Une première butée 50 d'immobilisation axiale est formée par l'extrémité libre d'une languette axiale 51 ménagée dans le fourreau 34. Cette languette 51 est déformable élastiquement radialement. La seconde butée 52 d'immobilisation axiale est ménagée également dans le fourreau 34.

De préférence, le dispositif de sécurité 33 comprend une paire de saillies radiales 48, diamétralement opposées, chaque saillie 48 étant destinée à coopérer avec une paire de butées d'immobilisation axiale 50, 52.

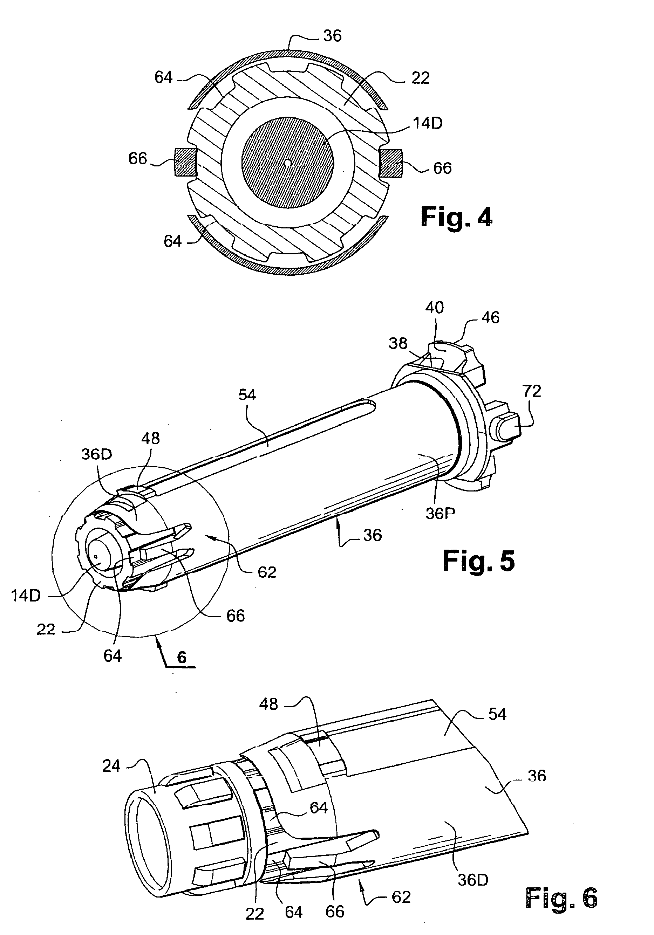

On notera que chaque butée 50 d'immobilisation axiale participe au guidage axial du support 36 de seringue par rapport au fourreau 34 en coopérant avec une rainure axiale 54 ménagée dans le support 36, clairement visible sur la figure 5.

Le ressort 42 est en appui, d'une part, sur un siège externe 56 ménagé sur le support 36 de seringue (voir figure 1), et d'autre part, sur un second siège interne 58 ménagé dans le fourreau 34 (voir figure 2). Ce second siège interne 58 est formé par le fond de la gorge 84 de logement du ressort 42 dans la position de dégagement de l'aiguille 28.

Le fourreau 34 porte des moyens externes de préhension destinés à être saisis par les doigts d'un utilisateur pour injecter le liquide par rapprochement axial de l'extrémité de manoeuvre 18P du poussoir et de ces moyens de préhension.

Dans l'exemple décrit, les moyens de préhension comprennent deux pattes radiales externes 60 solidaires du fourreau 34 (voir figure 1).

Le montage du dispositif de sécurité 33 puis de la seringue 12 dans le dispositif de sécurité 33 est réalisé de façon connue en soi, par exemple comme cela est proposé dans FR-A-2 830 765.

Par ailleurs, à l'exception de certains aspects liés à l'invention qui seront précisés par la suite, le fonctionnement du dispositif de sécurité 33 est classique, par exemple comme cela est décrit dans FR-A-2 830 765.

On rappellera ci-dessous les principales étapes de fonctionnement du dispositif de sécurité 33 en se référant aux figures 2 et 3.

Avant utilisation, l'ensemble 10 est dans une configuration d'attente tel que représenté sur la figure 2. Le capuchon 30 de protection de l'aiguille 28 est emmanché sur le porte-aiguille 26. Le fourreau 34 et le support 36 sont retenus dans leur position relative de dégagement de l'aiguille 28 par coopération des butées de retenue complémentaires 44,46.

Tout d'abord, l'utilisateur retire le capuchon 30 de façon à dégager l'aiguille 28.

Pour réaliser l'injection du liquide, l'utilisateur saisit l'ensemble 10 de façon à enfoncer le poussoir 18 dans le corps 12 de seringue en rapprochant axialement l'extrémité proximale 18P du poussoir des pattes de préhension 60.

L'utilisateur enfonce le poussoir 18 jusqu'à atteindre la position de fin d'injection représentée sur la figure 3.

Lors de l'enfoncement du poussoir 18, l'extrémité proximale 18P de ce poussoir, qui présente un contour C approprié, coopère avec les languettes élastiques 45 de façon à séparer l'une de l'autre les butées de retenue complémentaires 44, 46. Ces moyens de retenue étant libérés, le ressort 42 sollicite le support 36 de seringue de façon à placer les fourreau 34 et support 36 dans leur position relative d'escamotage de l'aiguille 28 tel que représenté sur la figure 3.

Dans la configuration de l'ensemble 10 représentée sur la figure 3, les saillies radiales 48 sont encliquetées entre les butées d'immobilisation axiales 50, 52 de façon à verrouiller les fourreau 34 et support 36 dans leur position d'escamotage de l'aiguille 28.

Conformément à l'invention, l'embout de fixation 22 et le support 36 de seringue comprennent des moyens complémentaires 62 de solidarisation en rotation entre eux autour de leurs axes.

Les moyens 62 de solidarisation en rotation comprennent par exemple, d'une part, un contour externe de l'embout de fixation 22 ayant une forme générale crénelée délimitant plusieurs rainures axiales 64 de solidarisation en rotation, réparties angulairement (voir notamment figures 4 à 6).

Les moyens 62 de solidarisation en rotation comprennent, d'autre part, deux cliquets 66 ou verrous de solidarisation en rotation, portés par le support 36 de seringue. Ces cliquets 66 sont diamétralement opposés.

En se référant notamment aux figures 5 et 6, on voit que, dans l'exemple décrit, chaque cliquet 66 est formé par une patte axiale venue de matière avec l'extrémité distale 36D du support de seringue et déformable élastiquement radialement. Cette patte formant cliquet 66 est rappelée élastiquement radialement, vers l'axe du support 36 de seringue, vers une position de coopération avec une rainure axiale complémentaire 64 de solidarisation en rotation. Ainsi, chaque cliquet 66 est rappelé élastiquement dans une position dans laquelle ce cliquet 66 coopère avec une rainure 64 formant des moyens de solidarisation en rotation portés par l'embout de fixation 22.

En variante, le support 36 de seringue pourrait ne comporter qu'un seul ou plus de deux cliquets 66 destinés à coopérer avec des moyens complémentaires de solidarisation en rotation portés par l'embout de fixation 22.

De préférence, le fourreau 34 comprend un dégagement radial 68 (voir notamment figures 1 et 7) qui, lorsque les fourreau 34 et support 36 sont dans leur position relative de dégagement de l'aiguille 28, tel que représenté sur les figures 1 et 2, est au droit radialement de la patte formant cliquet 66. Ce dégagement 68 autorise le déplacement radial de la patte formant cliquet 66 à l'encontre de sa force de rappel. La patte formant cliquet 66 est en effet déplacée à l'encontre de sa force de rappel, notamment lorsqu'elle est en appui sur une partie du contour de l'embout de fixation 22 séparant deux rainures 64, comme cela est représenté sur la figure 8.

De préférence, le dégagement radial 68 est formé par une fente ou rainure axiale ménagée dans l'extrémité distale 34D du fourreau.

Le fourreau 34 et le support 36 comprennent des moyens complémentaires 70 de solidarisation en rotation entre eux autour de leurs axes. Ces moyens 70 de solidarisation en rotation sont activés au moins lorsque les fourreau 34 et support 36 sont dans leur position relative de dégagement de l'aiguille 28 tel que représenté sur les figures 1 et 2.

Les moyens 70 forment également des moyens de retenue axiale du corps 14 de seringue par rapport au fourreau 34 lors de l'injection du liquide.

En se référant notamment aux figures 1 et 5, on voit que, dans l'exemple illustré, les moyens complémentaires 70 de solidarisation en rotation comprennent deux paires de saillie radiale 72 et rainure axiale 74 complémentaires ménagées sur les support 36 et fourreau 34.

Les deux paires de saillie radiale 72 et rainure axiale 74 complémentaires sont diamétralement opposées.

Suivant une variante, le dispositif de sécurité 33 pourrait ne comporter qu'une seule paire de saillie radiale 72 et rainure axiale 74.

Suivant une autre variante, les moyens 70 de solidarisation en rotation pourraient comprendre une saillie axiale, ménagée sur le support 36, coopérant avec une forme complémentaire, ménagée sur le fourreau 34, ou encore des méplats complémentaires ménagés sur le support 36 et le fourreau 34.

La saillie radiale 72 est, de préférence, ménagée sur l'extrémité proximale 36P du support de seringue. La rainure axiale complémentaire 74 est formée par une fente axiale ménagée dans l'extrémité proximale 34P du fourreau.

Les moyens de solidarisation en rotation 62 et 70 permettent d'immobiliser en rotation l'embout de fixation 22 par rapport au fourreau 34. En effet, lors du montage de la seringue 12 dans le dispositif de sécurité 33, généralement, le bouchon 24 est vissé sur l'embout de fixation 22. Deux cas peuvent alors se présenter.

Dans un premier cas, la position angulaire relative de l'embout de fixation 22 et du support 36 de seringue lors du montage est telle que les cliquets 66 s'emboîtent dans des rainures correspondantes 64, ceci automatiquement sous l'effet du rappel élastique de ces cliquets 66, comme cela est représenté sur les figures 4 à 7. L'embout de fixation 22 est alors solidaire en rotation du support 36 de seringue. Par ailleurs, les fourreau 34 et support 36 étant dans leur position relative de dégagement de l'aiguille, ils sont solidarisés en rotation entre eux par les moyens 72. L'embout de fixation 22 est donc solidaire en rotation, par transitivité, du fourreau de protection 36. Ainsi, un utilisateur saisissant le fourreau 34 peut facilement dévisser le bouchon 24 porté par l'embout de fixation 22 puis visser le porte-aiguille 26 sur cet embout de fixation 22 pour obtenir un ensemble 10 dans une configuration telle que représentée sur la figure 2. Ces opérations de vissage et dévissage sont faciles à réaliser car, dans les conditions habituelles d'utilisation, il n'y a pas de rotation intempestive de l'embout de fixation 22 autour du corps 14 de la seringue.

Dans le second cas, à la suite du montage de la seringue 12 dans le dispositif de sécurité 33, les cliquets 66 sont chacun intercalés entre deux rainures 64, comme cela est représenté sur la figure 8. Il suffit alors d'imposer une courte rotation à l'embout de fixation 22, par exemple par l'intermédiaire du bouchon 24 (qui comporte des moyens d'agrippage) pour provoquer l'emboîtement des cliquets 66 dans des rainures 64 correspondantes et ainsi se retrouver dans le premier cas décrit ci-dessus.

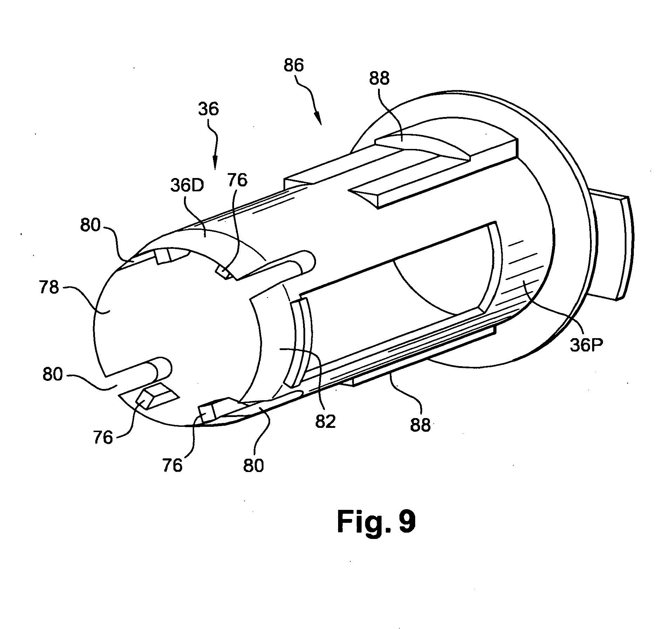

Dans une première variante de l'invention illustrée sur la figure 9, le support 36 de seringue porte au moins un cliquet 76 formé par une saillie axiale ménagée sur une surface interne 78 de l'extrémité distale 36D du support 36. Comme cela est illustré sur cette figure 9, dans cette variante, l'extrémité distale 36D du support 36 est munie de fentes axiales 80 permettant une déformation radiale élastique de l'extrémité distale 36D. L'extrémité distale 36D comprend dans cet exemple quatre fentes axiales 80 délimitant quatre portions 82 sensiblement égales et le support 36 porte deux paires de cliquets 76, réparties sur deux portions 82 diamétralement opposées.

Du fait que l'extrémité distale 36D est déformable radialement et élastiquement, il est possible de faire varier de manière élastique le diamètre de cette extrémité 36D. Ainsi, chaque cliquet 76 porté par les portions 82 de cette extrémité 36D, peut être rappelé élastiquement radialement, vers l'axe du support 36 de seringue, vers une position de coopération avec une rainure axiale complémentaire 64 formant des moyens de solidarisation en rotation portés par l'embout de fixation 22. Bien entendu, la répartition angulaire des cliquets 76 sur la surface interne 78 de l'extrémité 36D est choisie de manière à ce que les cliquets 76 puissent coopérer conjointement avec les rainures axiales complémentaires 64 portées par l'embout de fixation 22.

On notera que l'on peut adapter la forme, le nombre et l'agencement des différents cliquets exposés ci-dessus de façon à les adapter à l'embout de fixation avec lequel ils sont destinés à coopérer. Un avantage est de pouvoir adapter le dispositif à différents types de seringues et embouts.

Dans une autre variante, comme cela est illustré sur la figure 9, le dispositif de sécurité peut comprendre des troisièmes moyens complémentaires 86 de solidarisation en rotation du support 36 par rapport au fourreau 34 autour de leurs axes respectifs. Plus précisément, ces troisièmes moyens 86 sont activés au moins lors du déplacement axial des fourreau 34 et support 36 l'un par rapport à l'autre depuis la position de dégagement de l'aiguille 28 de seringue vers celle d'escamotage de cette aiguille 28.

Par exemple, ces troisièmes moyens complémentaires 86 comprennent au moins une nervure axiale 88 en forme générale de méplat portée par le support 36, s'étendant axialement dans une partie supérieure du support 36, comprenant l'extrémité proximale 36P. Cette nervure 88 est destinée à coopérer avec une encoche axiale (non représentée) de forme générale complémentaire ménagée dans une paroi interne latérale de la gorge annulaire 84 de logement du ressort 42. De préférence, les troisièmes moyens 86 comprennent deux nervures diamétralement opposées et deux encoches complémentaires. Grâce à la présence de ces troisièmes moyens 86, lors d'une phase d'amorce de la détente du ressort 42, toute rotation intempestive de ce dernier peut être évitée.

On notera que les troisièmes moyens 86 peuvent prendre la forme, comme dans cette autre variante exposée ci-dessus, d'une nervure axiale 88 portée par le support 36, ou bien comme sur la figure 2, d'une rainure 54 ménagée dans le support 36. Dans le cas de la nervure axiale 88, on notera que le guidage est particulièrement robuste et ne risque pas de provoquer un désengagement de la nervure 88 hors de l'encoche de la gorge 84 lors du déplacement axial relatif des support 36 et fourreau 34 de la position de dégagement de l'aiguille 28 de seringue vers celle d'escamotage de cette aiguille 28.

De préférence, l'ensemble 10 sera remis à l'utilisateur dans une configuration telle que les cliquets 66, 76 sont emboîtés dans des rainures 64 complémentaires. Au besoin, on prévoira, lors du montage de la seringue 12 dans le dispositif de sécurité 33, une brève opération de rotation de l'embout de fixation 22, de préférence en actionnant le bouchon 24 dans son sens de vissage dans l'embout 22, de façon à assurer l'emboîtement des cliquets 66 dans les rainures 64 correspondantes. Toutefois, si, lors de l'utilisation de l'ensemble 10, les cliquets 66 ne se trouvaient pas emboîtés dans les rainures 64, l'utilisateur, souhaitant dévisser le bouchon 24 pour le remplacer par le porte-aiguille 26, provoquera une rotation suffisante de l'embout de fixation 22 pour faire coïncider les cliquets 66 avec les rainures 64. L'utilisateur de l'ensemble 10 n'a donc pas à prévoir un geste spécifique, autre que le dévissage du bouchon 24 nécessaire pour son remplacement par le porte-aiguille 26, pour immobiliser en rotation l'embout de fixation 22 par rapport au fourreau 34.

L'invention ne se limite pas au mode de réalisation décrit ci-dessus.

En particulier, la forme générale crénelée du contour externe de l'embout 22 peut être délimitée par des formes variées autres que des rainures axiales, par exemple des rainures à bords non parallèles, à fond incliné, à extrémités ouvertes ou fermées, etc.

Les cliquets peuvent avoir des formes variées complémentaires du contour crénelé de l'embout 22.

On comprendra que les différentes variantes décrites peuvent être combinées, ou non, avec l'ensemble des fonctionnalités décrites dans tout ce qui précède.

## Revendications

1. Dispositif de sécurité pour une seringue d'injection de liquide comprenant un corps (14) de forme générale tubulaire formant un réservoir pour le liquide sur lequel est rapporté un embout (22) de forme générale annulaire pour la fixation d'un porte-aiguille (26) sur le corps (14) de seringue,
le dispositif étant du type comportant :
- un premier organe de forme générale tubulaire, appelé fourreau de protection (34), et
- un second organe de forme générale tubulaire, appelé support (36) de seringue, logé dans le fourreau (34) sensiblement coaxialement à ce dernier,
les fourreau (34) et support (36) étant déplaçables axialement l'un par rapport à l'autre entre deux positions dites de dégagement de l'aiguille (28) de seringue et d'escamotage de cette aiguille (28),
- le fourreau de protection (34) et le support (36) de seringue comprenant des seconds moyens complémentaires (70) de solidarisation en rotation entre eux autour de leurs axes, ces seconds moyens de solidarisation en rotation (70) étant activés au moins lorsque les fourreau (34) et support (36) sont dans leur position relative de dégagement de l'aiguille,
**caractérisé en ce que** :
- le support (36) de seringue comprend des premiers moyens (62) de solidarisation en rotation autour de son axe comprenant au moins un cliquet (66) rappelé élastiquement dans une position dans laquelle ce cliquet (66) est destiné à coopérer avec des moyens complémentaires (64) de solidarisation en rotation portés par l'embout de fixation (22).

2. Dispositif selon la revendication 1, dans lequel le support (36) de seringue porte au moins deux cliquets (66) de solidarisation en rotation, diamétralement opposés.

3. Dispositif selon la revendication 1 ou 2, dans lequel le cliquet (66) est formé par une patte axiale (66) venue de matière avec le support (36) de seringue rappelée élastiquement radialement vers l'axe de ce support (36) de seringue, destinée à coopérer avec une rainure complémentaire (64) de solidarisation en rotation ménagée sur le contour de l'embout de fixation (22).

4. Dispositif selon la revendication 3, dans lequel le fourreau (34) comprend au moins un dégagement radial (68) qui, lorsque les fourreau (34) et support (36) sont dans leur position relative de dégagement de l'aiguille (28) de seringue, est au droit radialement de la patte axiale (66) de solidarisation en rotation pour autoriser le déplacement radial de cette patte (66) à l'encontre de sa force de rappel.

5. Dispositif selon la revendication 4, dans lequel, la patte axiale (66) de solidarisation en rotation étant portée par une extrémité distale (36D) du support de seringue, le dégagement radial (68) est formé par une fente axiale ménagée dans une extrémité distale (34D) du fourreau.

6. Dispositif selon l'une quelconque des revendications précédentes, dans lequel les seconds moyens complémentaires (70) de solidarisation en rotation comprennent au moins une paire de saillie (72) et rainure (74) complémentaires ménagées sur les support (36) de seringue et fourreau de protection (34).

7. Dispositif selon la revendication 6, dans lequel la saillie (72) des seconds moyens de solidarisation en rotation (70) est ménagée sur une extrémité proximale (36P) du support de seringue et la rainure (74) des seconds moyens de solidarisation en rotation (70) est formée par une fente ménagée dans une extrémité proximale (34P) du fourreau de protection.

8. Dispositif selon la revendication 6 ou 7, dans lequel les seconds moyens complémentaires (70) de solidarisation en rotation comprennent deux paires de saillie (72) et rainure (74) complémentaires diamétralement opposées.

9. Dispositif selon l'une quelconque des revendications précédentes, comprenant des troisièmes moyens complémentaires (86) de solidarisation en rotation du support (36) par rapport au fourreau (34) autour de leurs axes, ces troisièmes moyens (86) étant activés au moins lors du déplacement axial des fourreau (34) et support (36) l'un par rapport à l'autre de la position de dégagement de l'aiguille (28) de seringue vers celle d'escamotage de cette aiguille (28).

10. Ensemble à seringue, du type comprenant :
- une seringue (12) d'injection de liquide comprenant un corps (14) de forme générale tubulaire formant un réservoir pour le liquide sur lequel est rapporté un embout (22) de forme générale annulaire pour la fixation d'un porte-aiguille (26) sur le corps (14) de seringue, et
- un dispositif de sécurité (33) pour la seringue,
**caractérisé en ce que** le dispositif de sécurité (33) est selon l'une quelconque des revendications précédentes.

11. Ensemble selon la revendication 10 comprenant un dispositif de sécurité (33) selon l'une quelconque des revendications 2 à 4, dans lequel le contour de l'embout de fixation (22) à une forme générale crénelée délimitant plusieurs rainures axiales (64) de solidarisation en rotation réparties angulairement.

12. Ensemble selon la revendication 10 ou 11, dans lequel l'embout de fixation (22) est destiné à coopérer par vissage avec un bouchon (24) d'obturation de la seringue ou le porte-aiguille (26).

## Claims

1. A safety device for a liquid injection syringe comprising a body (14) of generally tubular shape forming a reservoir for the liquid and having fitted thereon an endpiece (22) of generally annular shape for fastening a needle carrier (26) on the syringe body (14), the device being of the type comprising:
· a first member of generally tubular shape, referred to as a protective sheath (34); and
· a second member of generally tubular shape, referred to a syringe support (36), received inside the sheath (34), substantially coaxially therewith;
the sheath (34) and the support (36) being movable axially relative to each other between two positions, namely a disengaged position of the syringe needle (28), and a retracted position of said needle (28);
· the protective sheath (34) and the syringe support (36) including second complementary anti-rotation means (70) for constraining them in rotation relative to each other about their axes, these second anti-rotation means (70) being activated at least when the sheath (34) and the support (36) are in their relative disengaged position of the needle,
the device being **characterized in that**:
• the syringe support (36) includes first anti-rotation means (62) for constraining rotation about its axis and comprising at least one pawl (66) urged resiliently into a position in which the pawl (66) is designed to co-operate with complementary anti-rotation means (64) carried by the fastener endpiece (22).

2. A device according to claim 1, wherein the syringe support (36) carries at least two diametrically-opposite anti-rotation pawls (66).

3. A device according to claim 1 or claim 2, wherein the pawl (66) is formed by an axial tab (66) molded integrally with the syringe support (36) and urged resiliently radially towards the axis of said syringe support (36), being designed to co-operate with a complementary anti-rotation groove (64) formed in the outline of the fastener endpiece (22).

4. A device according to claim 3, wherein the sheath (34) includes at least one radial setback (68) that is positioned, when the sheath (34) and the support (36) are in their relative disengaged position of the syringe needle (28), to be radially in register with the anti-rotation axial tab (66) so as to allow said tab (66) to move radially against its return force.

5. A device according to claim 4, wherein the axial anti-rotation tab (66) is carried by a distal end (36D) of the syringe support, and the radial setback (68) is formed by an axial slot provided in a distal end (34D) of the sheath.

6. A device according to any preceding claim, wherein the second complementary anti-rotation means (70) comprise at least one complementary pair constituted by a projection (72) and a groove (74) provided on or in the syringe support (36) and the protective sheath (34).

7. A device according to claim 6, wherein the projection (72) of the second anti-rotation means (70) is provided on a proximal end (36P) of the syringe support, and the groove (74) of the second anti-rotation means (70) is formed by a slot provided in a proximal end (34P) of the protective sheath.

8. A device according to claim 6 or claim 7, wherein the second complementary anti-rotation means (70) comprise two diametrically-opposite complementary pairs, each comprising a projection (72) and a groove (74).

9. A device according to any preceding claim, including third complementary anti-rotation means (86) for constraining the support (36) and the sheath (34) in rotation relative to each other about their axes, these third means (86) being activated at least during axial displacement of the sheath (34) and the support (36) relative to each other from the disengaged position of the syringe needle (38) towards the retracted position of said needle (28).

10. A syringe assembly of the type comprising:
• a liquid injection syringe (12) comprising a body (14) of generally tubular shape forming a reservoir for the liquid and having fitted thereon an endpiece (22) of generally annular shape for fastening a needle carrier (26) to the syringe body (14); and
• a safety device (33) for the syringe;
the syringe assembly being **characterized in that** the safety device (33) is in accordance with any preceding claim.

11. An assembly according to claim 10, including a safety device (33) according to any one of claims 2 to 4, in which the outline of the fastener endpiece (22) is of generally crenellated shape defining a plurality of angularly distributed anti-rotation axial grooves (64).

12. An assembly according to claim 10 or claim 11, wherein the fastener endpiece (22) is designed to co-operate by screw-fastening with a plug (24) for closing the syringe or the needle carrier (26).

## Patentansprüche

1. Sicherheitsvorrichtung für eine Injektionsspritze zum Injizieren von Flüssigkeiten, die ein allgemeinen röhrenförmiges Gehäuse (14) umfasst, das ein Reservoir für die Flüssigkeit bildet, an dem ein allgemeinen ringförmiges Ansatzstück (22) zum Befestigen eines Nadelhalters (26) an dem Gehäuse (14) der Spritze angebracht ist, wobei die Vorrichtung generell umfasst:
ein erstes allgemein röhrenförmiges Element, genannt Schutzhülle (34), und
ein zweites allgemein röhrenförmiges Element, genannt Spritzenträger (36), der in der Hülle (34) im wesentlichen koaxial zu dieser angeordnet ist,
wobei die Hülle (34) und der Träger (36) axial zueinander zwischen zwei Positionen beweglich sind, nämlich zwischen dem Auslösen der Nadel (28) der Spritze und dem Einziehen der Nadel (28),
wobei die Schutzhülle (34) und der Träger (36) der Spritze zweite komplementäre Mittel (70) zur drehfesten Verbindung der beiden um ihre Achsen umfasst, wobei die zweiten drehfesten Verbindungsmittel (70) wenigstens dann aktiviert sind, wenn sich die Hülle (34) und der Träger (36) in ihrer Position zum Auslösen der Nadel befinden,
**dadurch gekennzeichnet, dass**:
der Träger (36) der Spritze erste Mittel (62) für eine drehfeste Verbindung um seine Achse mit wenigstens einer Klinke (66) umfasst, die elastisch in eine Position gedrückt wird, in welcher die Klinke (66) dazu bestimmt ist, mit den komplementären drehfesten Verbindungsmitteln (64) zusammenzuwirken, die von dem Befestigungsansatzstück (22) gehalten werden.

2. Vorrichtung nach Anspruch 1, bei der der Träger (36) der Spritze wenigstens zwei diametral gegenüberliegende Klinken (66) für die drehfeste Verbindung umfasst.

3. Vorrichtung nach Anspruch 1 oder 2, bei der die Klinke (66) durch eine Axialzunge (66) gebildet wird, die einstückig mit dem Träger (36) der Spritze verbunden ist, elastisch radial gegen die Achse des Trägers (36) der Spritze drückt und dazu bestimmt ist, mit einer komplementären Nut (64) für die drehfeste Verbindung zusammenzuwirken, die im Umfang des Befestigungsansatzstückes (22) vorgesehen ist

4. Vorrichtung nach Anspruch 3, bei der die Hülle (34) wenigstens einen Radialauslöser (68) umfasst, der, wenn sich die Hülle (34) und der Träger (36) in ihrer Position zum Auslösen der Nadel (28) der Spritze befinden, radial senkrecht zu der Axialzunge (66) für die drehfeste Verbindung steht, um die radiale Bewegung dieser Zunge (66) gegen ihre Rückholkraft zu ermöglichen.

5. Vorrichtung nach Anspruch 4, bei der die Axialzunge (66) für die drehfeste Verbindung von einem äußeren Ende (36D) des Trägers der Spritze gehalten wird, wobei der Radialauslöser (68) durch einen Axialschlitz gebildet wird, der an einem äußeren Ende (34D) der Hülle vorgesehen ist.

6. Vorrichtung nach einem der vorangehenden Ansprüche, bei der die zweiten komplementären drehfesten Verbindungsmittel (70) wenigstens ein Paar Vorsprünge (72) und Nuten (74) umfassen, die komplementär an dem Träger (36) der Spritze und der Schutzhülle (34) vorgesehen sind.

7. Vorrichtung nach Anspruch 6, bei der der Vorsprung (72) der zweiten drehfesten Verbindungsmittel (70) an einem diesseitigen Ende (36P) des Trägers der Spritze vorgesehen ist und die Nut (74) der zweiten drehfesten Verbindungsmittel (70) durch einen Schlitz gebildet wird, der an einem diesseitigen Ende (34P) der Schutzhülle vorgesehen ist.

8. Vorrichtung nach Anspruch 6 oder 7, bei der die zweiten komplementären drehfesten Verbindungsmittel (70) zwei Paare von diametral gegenüberliegenden komplementären Vorsprüngen (72) und Nuten (74) umfassen.

9. Vorrichtung nach einem der vorangehenden Ansprüche, die dritte komplementäre Mittel (86) für die drehfeste Verbindung des Trägers (36) in Bezug auf die Hülle (34) um ihre Achsen aufweist, wobei die dritten Mittel (86) wenigstens bei der axialen Bewegung der Hülle (34) und des Trägers (36) zueinander aus der Auslöseposition der Nadel (28) der Spritze in die Einziehposition dieser Nadel (28) aktiviert sind.

10. Spritzenaufbau, der umfasst:
eine Spritze (12) zum Injizieren von Flüssigkeiten mit einem allgemein röhrenförmigen Gehäuse (14), das ein Reservoir für die Flüssigkeit bildet, an welchem ein allgemein ringförmiges Ansatzstück (22) zur Befestigung eines Spritzenträgers (26) an dem Gehäuse (14) der Spritze angebracht ist, und
eine Sicherheitseinrichtung (33) für die Spritze,
**dadurch gekennzeichnet, dass** die Sicherheitseinrichtung (33) eine nach einem der vorangehenden Ansprüche ist.

11. Aufbau nach Anspruch 10, der eine Sicherheitseinrichtung (33) nach einem der Ansprüche 2 bis 4 umfasst, bei der der Umfang des allgemein gezahnten Befestigungsansatzstückes (22) mehrere axiale, winkelmäßig verteilte Nuten (64) für eine drehfeste Verbindung umgrenzt.

12. Aufbau nach Anspruch 10 oder 11, bei dem das Befestigungsansatzstück (22) dazu bestimmt ist, über Verschraubung mit einem Stopfen (24) zum Verschließen der Spritze oder des Nadelträgers (26) zusammenzuwirken.
